Europäisches Patentamt

European Patent Office

Office européen des brevets

(11) Numéro de publication : **0 465 361 B1**

# FASCICULE DE BREVET EUROPEEN

(45) Date de publication du fascicule du brevet :
25.01.95 Bulletin 95/04

(51) Int. Cl.⁶ : **H05G 1/46, A61B 6/00**

(21) Numéro de dépôt : **91401836.1**

(22) Date de dépôt : **03.07.91**

(54) **Procédé de détermination de la fonction représentant l'effet de non réciprocité d'un film radiographique.**

(30) Priorité : **06.07.90 FR 9008626**

(43) Date de publication de la demande :
**08.01.92 Bulletin 92/02**

(45) Mention de la délivrance du brevet :
**25.01.95 Bulletin 95/04**

(84) Etats contractants désignés :
**DE IT NL**

(56) Documents cités :
**EP-A- 0 208 607**
**WO-A-87/01555**
**GB-A- 2 004 437**

(73) Titulaire : **GENERAL ELECTRIC CGR S.A.**
**100, rue Camille-Desmoulins**
**F-92130 Issy les Moulineaux (FR)**

(72) Inventeur : **Heidsieck, Robert**
**Cabinet BALLOT-SCHMIT,**
**7 rue Le Sueur**
**F-75116 Paris (FR)**

(74) Mandataire : **Ballot, Paul Denis Jacques et al**
**Cabinet Ballot-Schmit,**
**7, rue Le Sueur**
**F-75116 Paris (FR)**

EP 0 465 361 B1

## Description

L'invention concerne les systèmes de radiologie utilisant un film radiographique et, plus particulièrement dans de tels systèmes, un procédé qui permet de déterminer l'effet de non réciprocité du film radiographique.

Un système de radiologie comprend essentiellement un tube à rayons X et un récepteur d'un tel rayonnement entre lesquels est interposé l'objet à examiner tel qu'une partie du corps d'un patient. Le récepteur image qui est, par exemple, un couple film-écran, fournit après une durée de pose appropriée et développement du film, une image de l'objet. Pour que l'image de l'objet puisse être exploitée dans les meilleures conditions, il faut que les différents points qui constituent cette image présentent entre eux un contraste suffisant, c'est-à-dire que le noircissement du film radiographique soit correct, et cela d'une radiographie à la suivante, malgré les différences d'opacité que peut présenter l'objet radiographié.

Le noircissement du film est lié à la quantité d'énergie du rayonnement incident sur le couple film-écran, c'est-à-dire le produit de l'intensité du rayonnement auquel est soumis le film radiographique, ou débit de dose "film", par le temps durant lequel le film est exposé à ce rayonnement. En conséquence, pour obtenir un noircissement constant du film d'une radiographie à la suivante, il est connu de mesurer, au cours de l'examen, l'énergie incidente sur le film au moyen d'une cellule de détection placée en général avant le récepteur qui est sensible au rayonnement X et qui fournit un courant proportionnel au débit de dose "film". Ce courant est intégré, à compter du début de la pose, dans un circuit intégrateur qui fournit une valeur croissante au cours de la pose. Cette valeur croissante est comparée durant le temps de pose à une valeur de consigne fixe, préalablement établie en fonction des caractéristiques du film. La fin du temps de pose est déterminée par l'instant auquel la comparaison indique que la valeur représentative de énergie incidente sur le film est égale à la valeur de consigne.

Dans le cas où le film radiographique est directement soumis au rayonnement X et que la variation des temps de pose d'un examen à l'autre est suffisamment faible, un noircissement constant du film est obtenu d'une pose à la suivante, indépendamment de la durée du temps de pose S, à condition que le produit du temps de pose S par le débit de dose F soit constant, c'est-à-dire que la valeur résultant de l'intégration doit rester constante. Ceci n'est vrai que si les caractéristiques du film obéissent à la loi de réciprocité qui indique que la densité optique du film est proportionnelle au produit F x S et si la réponse du film est indépendante de la qualité du faisceau de rayons X incident.

Cette loi de réciprocité n'est plus vérifiée dans le cas où la variation des temps de pose est forte.

Par ailleurs, dans le cas où le film radiographique est associé à un écran renforçateur, le noircissement du film dépend de la qualité du spectre. En effet, la réponse de l'écran dépend de la répartition énergétique du spectre du rayonnement reçu, ce qui signifie qu'il est sensible au durcissement de spectre et au changement de tension du tube à rayons X.

Enfin, il existe certaines applications pour lesquelles il est pénalisant que la cellule de détection soit placée avant le film, par exemple en mammographie, car l'énergie de rayonnement est telle que la cellule de détection serait alors visible sur le film. Dans ce cas, elle est placée derrière le récepteur image mais cela crée une difficulté supplémentaire car le signal perçu par la cellule détectrice est celui qui n'a pas contribué au noircissement du film. Il en résulte que la mesure effectuée par la cellule de détection ne représente pas, en général, la lumination incidente sur le film radiographique.

L'écart à la loi de réciprocité, qui varie selon le type de film, représente la variation relative de la lumination nécessaire pour obtenir une densité optique constante lorsque le temps de pose S varie alors que le spectre du rayonnement X est constant. Ceci se traduit par le fait que pour obtenir une même densité optique du film, la lumination devra être par exemple de 1 pour un temps de pose S = 0,1 seconde, de 1, 3 pour S = 1 seconde et de 2 pour S = 4 secondes.

Cet écart à la loi de réciprocité est dû au phénomène connu sous le nom d'effet Schwarzschild. Cet effet est décrit notamment dans le livre intitulé : "CHIMIE ET PHYSIQUE PHOTOGRAPHIQUES" de Pierre GLAFKIDES - 4ème édition, pages 234 à 238 et édité par PUBLICATIONS PHOTO-CINEMA Paul MONTEL.

Pour tenir compte de cet écart à la loi de réciprocité, il a été proposé différentes solutions et l'une d'entre elles a été décrite dans le brevet français 2 584 504 (EP-A-0208607). Dans ce brevet, il est prévu de comparer la valeur intégrée du signal fourni par la cellule de détection à une valeur de consigne qui varie au cours de la pose selon une loi déterminée. Plus précisément, à partir du début de chaque temps de pose, on ajoute à la différence des valeurs du signal intégré et de la consigne une valeur additionnelle qui est croissante en fonction du temps selon une loi préalablement déterminée, par exemple exponentielle.

Cette loi préalablement déterminée, qu'elle soit exponentielle ou autre, ne rend compte de l'écart à la loi de réciprocité que de manière imparfaite, notamment en ne tenant pas compte des variations de l'intensité lumineuse effectivement reçue par le film. En outre, cette correction ne tient pas compte des effets d'autres phénomènes tels que le durcissement du rayonnement X dû à l'épaisseur d'objet traversé, à la modification du spectre due à la tension du tube à rayons X.

De plus, dans ce procédé, la cellule de détection est placée avant le récepteur image.

Une autre solution qui tient compte des différents effets qui interviennent, notamment les variations du courant du tube, le durcissement du spectre dû à l'épaisseur d'objet traversé, la modification du spectre due à la tension de tube et, lors de la présence d'un écran renforçateur, la réponse en absorption de celui-ci, a été décrite dans la demande de brevet déposée ce jour (EP-A-0465360) et intitulée : "PROCEDE DE DETERMINATION AUTOMATIQUE DE LA DUREE D'EXPOSITION D'UN FILM RADIOGRAPHIQUE ET SYSTEME DE MISE EN OEUVRE".

Dans ce procédé, pour tenir compte des variations du courant du tube à rayons X et, plus généralement, des variations du débit photonique sur le film radiographique, on utilise un coefficient de non réciprocité CNRD exprimé en débit photonique incident sur le film par des mesures et calculs particuliers.

Le but de la présente invention est donc de mettre en oeuvre un procédé de détermination de la fonction représentant l'effet de non réciprocité d'un film radiographique exprimée en fonction du débit photonique sur le film radiographique.

L'invention concerne un procédé de détermination de la fonction représentant l'effet de non réciprocité d'un film radiographique dans un système de radiologie selon la revendication 1.

Les coefficients CNRD (d) peuvent être modélisés suivant une fonction telle que :

$$\text{CNRD (d)} = A'_0 + A'_1 \log 1/d + A'_2 [\log 1/d]^2 \quad (20)$$

dont les paramètres $A'_0$, $A'_1$ et $A'_2$ sont estimés à partir des valeurs $d_i$ et $\text{CNRD}(d_i)$.

L'opération (a) du procédé selon la revendication 1 peut comprendre les opérations suivantes :

($a_1$) la modification du courant de chauffage du tube de manière à obtenir différentes valeurs dudit courant;

($a_2$) le relevé des valeurs M ($t_i$) fournies par le circuit intégrateur pour différents temps de pose ($t_i$) de manière à obtenir une densité optique $D0_{refo}$ du film fixée;

($a_3$) le calcul du rapport

$$\frac{M (t_i)}{M (t_{ref})} \quad (29)$$

qui donne le coefficient CNRT ($t_i$) avec M ($t_{ref}$) la valeur M ($t_i$) pour $t_i = t_{ref}$

L'opération (a) du procédé peut également être réalisée par les opérations suivantes :

(g1) réalisation à l'aide d'un sensitographe à temps variable, d'un premier sensitogramme $S_{refo}$ lorsque le temps de pose est réglé pour un temps de référence $t_{refo}$;

(g2) réalisation à l'aide du même sensitographe à temps variable, de q sensitogrammes $S_1$ à $S_q$ pour q temps de pose différents $t_i$;

(g3) choix d'une densité optique de référence $DO_{refo}$,

(g4) mesure sur chaque sensitogramme, de l'échelon d'éclairement $Ech_{refo}$, $Ech_1...Ech_i...Ech_q$ correspondant à la densité optique $DO_{refo}$

(g5) calcul des coefficients CNRT ($t_i$) par l'équation :

$$\text{CNRT } (t_i) = \exp\left[\log_{10}\left[\frac{Ech_{refo} - Ech_i}{K}\right]\right] \quad (28)$$

avec $K = 2/\log_{10}(2)$

Les coefficients CNRT ($t_i$), peuvent être modélisés sous la forme d'un modèle analytique :

$$\text{CNRT (t)} = A_0 + A_1 \log t + A_2 [\log t]^2 \quad (18)$$

dont les paramètres $A_0$, $A_1$ et $A_2$ sont estimés à partir des valeurs $t_i$ et $\text{CNRT}(t_i)$.

Pour l'opération (b) du procédé selon la revendication 1 la lumination de référence $L_{ref}$ peut être déterminée par divers étalonnages, par exemple à l'aide d'une cellule détectrice qui mesure la lumière émise par l'écran.

D'autres buts, caractéristiques et avantages de la présente invention apparaîtront à la lecture de la description suivante du procédé selon l'invention et d'un exemple particulier de réalisation du système de radiologie pour le mettre en oeuvre, ladite description étant faite en relation avec les dessins joints dans lesquels :

- la figure 1 est un schéma fonctionnel d'un système de radiologie qui permet de mettre en oeuvre le procédé selon l'invention;

- la figure 2 est un diagramme montrant des courbes obtenues en mettant en oeuvre un procédé d'étalonnage utilisé dans le procédé selon l'invention,

- la figure 3 est un diagramme montrant une courbe de variation des coefficients de non réciprocité CNRT en fonction du temps de pose t,

EP 0 465 361 B1

- la figure 4 est un diagramme montrant une courbe de variation des coefficients de non réciprocité CNRD en fonction de l'inverse du débit d, et
- la figure 5 est un diagramme montrant des courbes de variation de la densité optique d'un film radiographique en fonction de la lumination.

Un système de radiologie auquel s'applique le procédé de détermination automatique du temps de pose d'un objet à radiographier 13 selon l'invention comprend une source 11 de rayonnement X tel qu'un tube à rayons X qui fournit un faisceau 14 de rayons X illuminant cet objet 13 et un récepteur image 17, tel qu'un couple film-écran, qui est placé de manière à recevoir les rayons X ayant traversé ledit objet et qui fournit, après une durée de pose S appropriée et développement du film, une image de l'objet 13.

Pour mettre en oeuvre le procédé de l'invention, le système comprend, en outre, une cellule de détection 12 qui est placée derrière le récepteur image 17 dans le cas d'un film radiographique avec écran renforçateur. Cette cellule peut être placée devant le récepteur dans le cas d'un film sans écran renforçateur. La cellule de détection 12 permet de convertir une grandeur physique caractéristique du rayonnement X ayant traversé l'objet et le récepteur image, tel que le KERMA ou la fluence énergétique, en un signal de mesure L, par exemple de type électrique. Le signal L, fourni par la cellule détection 12, est appliqué à un circuit 16 qui réalise une intégration du signal électrique pendant la durée S de la pose. Le signal M, qui résulte de l'intégration, est une mesure du rayonnement ayant traversé l'objet 13 pendant la durée S de la pose.

La source 11 de rayonnement X est associée à un dispositif d'alimentation 15 qui fournit une haute tension d'alimentation variable $V_m$ du tube à rayons X et qui comprend un appareil de mesure du courant anodique I dudit tube. Afin de modifier la durée du temps de pose S, le dispositif d'alimentation 15 et le tube à rayons X comprennent des moyens pour démarrer l'émission de rayons X à un instant précis et l'arrêter après une durée S variable qui est déterminée en fonction du signal M fourni par le circuit 16 et des valeurs de I, S et $V_m$ et, plus précisément, du rapport M/I x S qui est appelé rendement D et qui est calculé par le dispositif 18. Les valeurs du rendement D sont traitées par un calculateur ou microprocesseur 19.

Le procédé selon l'invention sera maintenant décrit dans le cadre d'un procédé plus général qui est celui de la détermination automatique de la durée d'exposition d'un film radiographique, procédé qui fait l'objet d'une demande de brevet (EP-A- 0465360) déposée le même jour que la présente demande et intitulée : "PROCEDE DE DETERMINATION AUTOMATIQUE DE LA DUREE D'EXPOSITION D'UN FILM RADIOGRAPHIQUE ET SYSTEME DE MISE EN OEUVRE.

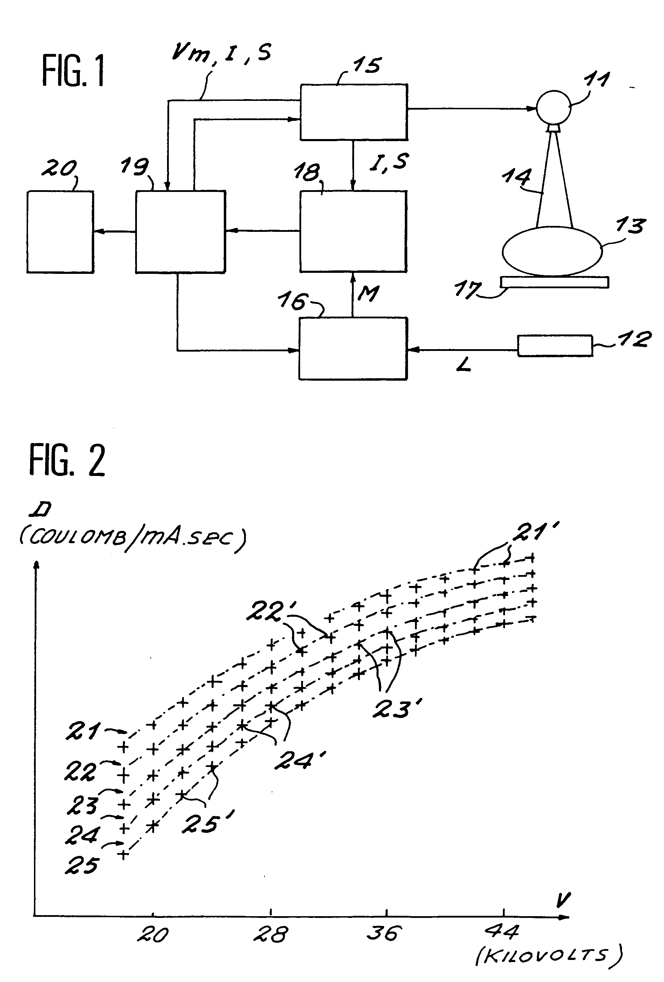

La première opération du procédé de détermination automatique de la durée d'exposition consiste à effectuer un étalonnage du système de radiologie de la figure 1, qui aboutit à une fonction d'estimation de la lumination vue par le film radiographique. Cet étalonnage et la fonction d'estimation sont décrits dans une demande de brevet (EP-A-0465362) déposée le même jour que la présente demande et intitulée : "PROCEDE D'ESTIMATION ET D'ETALONNAGE DE LA LUMINATION RECUE PAR UN FILM RADIOGRAPHIQUE".

Pour la compréhension de la suite de la description, on rappellera que le procédé d'estimation de la lumination reçue par un film radiographique est basé sur des étalonnages aboutissant à la définition d'une fonction proportionnelle au débit photonique sur le film, appelée débit-film, et sur un étalonnage permettant de faire le lien entre la fonction débit-film et la lumination reçue par le film dans des conditions de référence fixées et aboutissant à un noircissement donné du film. Ce dernier étalonnage sera plus amplement détaillé dans la suite de la description.

Les étalonnages qui permettent de définir la fonction débit-film sont dérivés d'un procédé d'étalonnage décrit dans la demande de brevet n° 89 07686 (correspondant à EP-A-0402244) déposée le 9 juin 1989 et intitulée "PROCEDE D'ETALONNAGE D'UN SYSTEME RADIOLOGIQUE ET DE MESURE DE L'EPAISSEUR EQUIVALENTE D'UN OBJET ". Ce procédé consiste à mesurer le rendement D de la cellule pour chaque étalon aux tensions d'alimentation $V_m$ choisies. Plus précisément, avec un premier étalon d'épaisseur $E_1$, on effectue une mesure de rendement $D_{1m}$ pour chaque valeur $V_m$ constituant un ensemble déterminé. Ces valeurs $D_{1m}$ en fonction de la tension $V_m$ peuvent être reportées sur un diagramme pour obtenir les points 21' de la figure 2.

Les mesures de rendement D sont effectuées pour un autre étalon d'épaisseur $E_2$ et on obtient les valeurs $D_{2m}$ correspondant aux points 22' de la figure 2 et ainsi de suite pour obtenir les autres séries de points 23', 24' et 25' correspondant respectivement aux rendements $D_{3m}$ $D_{4m}$ et $D_{5m}$ et aux épaisseurs $E_3$, $E_4$ et $E_5$.

Il est à noter que, sur la figure 2, les rendements $D_{pm}$ ont été reportés en ordonnées logarithmiques tandis que les tensions d'alimentation ont été reportées en abscisses de 20 kilovolts à 44 kilovolts.

Ces séries de points 21' à 25' servent à définir les paramètres d'un modèle analytique qui décrit le comportement du rendement D en fonction des paramètres $V_m$ et $E_p$ pour une configuration donnée du système radiologique. Ce modèle analytique sera noté :

$$D = f(V_m, E_p) \quad (1)$$

Les paramètres du modèle analytique peuvent être ajustés à l'aide d'outils classiques d'estimation tels

4

que la méthode de minimisation de l'erreur quadratique.

Les courbes 21 à 25 représentent la valeur du rendement D donnée par le modèle analytique représenté par l'expression :

$$D = f(V_m, E_p) = \exp[f_1(V_m) + E_p \times f_2(V_m)] \quad (2)$$

dans laquelle $f_1(V_m)$ et $f_2(V_m)$ sont des polynômes du deuxième degré dont l'expression est donnée par :

$$f_1(V_m) = A_o + A_1 V_m + A_2 V_m^2$$
$$f_2(V_m) = B_o + B_1 V_m + B_2 V_m^2$$

La fonction inverse de celle exprimée par la formule (2) permet de calculer $E_p$ si l'on connaît D et $V_m$ en utilisant la formule (3) suivante :

$$E_p = g(V_m, D) = \frac{\text{Ln}(D) - f_1(V_m)}{f_2(V_m)} \quad (3)$$

sachant que $f_2(V_m)$ ne peut pas s'annuler pour les valeurs courantes de $V_m$ car le rendement D dépend toujours de l'épaisseur $E_p$ aux tensions $V_m$ considérées. En d'autres termes, à un couple de valeurs $(E_p, V_m)$ correspond une mesure de rendement D, ce qui permet de déterminer $E_p$ en fonction de $V_m$ et D. Au cours d'un examen radiologique, une mesure de rendement D qui est effectuée avec une tension d'alimentation $V_m$ donnée permet de déterminer une épaisseur équivalente exprimée dans les unités utilisées pour $E_p$.

Cet étalonnage est effectué par deux fois avec des configurations différentes du système de radiologie en ce qui concerne le récepteur 17. Le premier de ces étalonnages est réalisé avec le récepteur 17 sans écran renforçateur. Suivant l'équation (1), on détermine une fonction f' qui donne lieu à des valeurs de rendement de la cellule 12 notées $D_{se}$ telles que :

$$D_{se} = f'(V_m, E_p) \quad (4)$$

et la fonction inverse :

$$E_p = g'(V_m, D_{se}) \quad (5)$$

La deuxième opération du procédé consiste à effectuer un deuxième étalonnage muni d'un écran renforçateur avec un récepteur 17 et on obtient alors une série de valeurs de rendement $D_c$ et on détermine, comme précédemment, la fonction f'' telle que :

$$D_c = f''(V_m, E_p) \quad (6)$$

et la fonction inverse

$$E_p = g''(V_m, D_c) \quad (7)$$

Des deux étalonnages précédents, on déduit une fonction $D_f$ qui représente le rendement sur le film tel que :

$$D_f = D_{se} - D_c$$

soit

$$D_f = f'(V_m, E_p) - f''(V_m, E_p) \quad (8)$$

Cette fonction $D_f$ ne tient pas compte de la modification du spectre du rayonnement X due à la filtration additionnelle entre l'écran renforçateur et la cellule de détection 12 qui provient, par exemple, de la face de sortie de la cassette contenant le couple film-écran. Pour en tenir compte, on remplace $E_p$ dans l'équation (8) par $(E_p - \text{sup.filtre})$ où sup.filtre est l'épaisseur équivalente à l'objet radiographié correspondant à cette filtration.

On obtient cette épaisseur équivalente en plaçant, par exemple, dans le faisceau 14 un objet équivalent à cette filtration et en utilisant la fonction étalonnée déterminant l'épaisseur équivalente g' ou g'' suivant la configuration de la machine.

Comme le produit $D_f \times I \times t$ est proportionnel à l'énergie absorbée dans l'écran renforçateur pendant un temps t et pour un courant anodique I, la quantité $D_f \times I$, notée débit-film, est proportionnelle au débit photonique incident sur le film et est exprimée dans les unités de mesure du signal de la cellule détectrice 12. Cette relation de proportionnalité est d'autant mieux vérifiée que le nombre de photons lumineux émis par l'écran renforçateur est lui-même proportionnel à l'énergie absorbée. Si le nombre de photons lumineux émis par l'écran répond à une autre loi en fonction de l'énergie absorbée, il faut appliquer cette autre loi sur $D_f \times I$ pour obtenir le "débit-film".

Un dernier étalonnage consiste à relier les fonctions électriques précédemment décrites à une valeur du noircissement du film, c'est-à-dire une densité optique que l'on souhaite obtenir à la fin de la pose. Le choix de cette valeur est effectué par le praticien en fonction du couple film-écran, du type de diagnostic, de la partie du corps du patient à examiner et de ses habitudes d'examen des radiographies. Ce choix permet de déterminer la lumination de référence, notée $L_{ref}$, c'est-à-dire la lumination que doit recevoir le film, dans des conditions de références fixées, pour arriver à un tel noircissement. Le procédé de détermination de $L_{ref}$ sera décrit ultérieurement.

Ces opérations d'étalonnage ne sont pas effectuées à chaque examen radiologique d'un objet ou d'un patient mais seulement une fois de temps à autre pour tenir compte des variations des caractéristiques du système de radiologie au cours du temps, notamment le vieillissement du tube à rayons X. Les résultats de ces opérations sont enregistrés dans la mémoire du microprocesseur 19 sous la forme des fonctions représentées par les équations 4 à 8, ce qui signifie que le microprocesseur 19 sait calculer $E_p$ s'il connaît $D_c$ et peut alors calculer $D_f$.

Au cours de l'examen radiologique du patient, le procédé consiste, en outre, à effectuer les opérations principales suivantes :

(e1) mise en place de l'objet ou du patient à radiographier,

(e2) déclenchement du début de la pose par le praticien

(e3) mesure du rendement $D_c$ un certain temps $t'$ après le début de la pose,

(e4) calcul de l'épaisseur équivalente à partir de la mesure de rendement $D_c$,

(e5) calcul du rendement $D_f$ au niveau du film,

(e6) estimation de la lumination reçue par le film depuis le début de la pose,

(e7) calcul de la lumination restant à acquérir pour obtenir le noircissement choisi,

(e8) calcul prévisionnel des mA.s restant à débiter dans le tube à rayons X pour obtenir le noircissement choisi,

(e9) mesure des mA.s, notée $mAs_{mes}$, débités suivant le cas depuis le début de la pose ou de la mesure précédente,

(e10) arrêt du rayonnement X lorsque les $mAs_{mes}$ sont supérieurs ou égaux aux mA.s calculés ou retour à l'opération (e3) dans le cas contraire.

Il est à noter que l'on appelle lumination le produit de la quantité de lumière reçue, par exemple l'éclairement EC de la surface sensible, par la durée de l'exposition ou pose.

L'opération (e3) consiste à mesurer la valeur intégrée D fournie par le dispositif 18 un certain temps $t'$ après le début de la pose sachant que l'intégrateur 16 a été remis à zéro soit, suivant le cas, au début de la pose, soit après la dernière mesure. Le temps $t'$ d'intégration correspond, suivant le cas, au temps écoulé depuis le début de la pose ou au temps écoulé depuis la dernière mesure.

L'opération (e4) est effectuée par le microprocesseur 19 à partir du premier étalonnage du système de radiologie tel que décrit ci-dessus : elle est régie par l'équation (7); on obtient alors une valeur $E_1$ de l'épaisseur équivalente.

Il est à remarquer que pour la deuxième itération du procédé et les suivantes, il n'est pas nécessaire d'effectuer l'opération (e4) dans la mesure où l'estimation de l'épaisseur équivalente a été suffisamment précise lors de la première itération.

L'opération (e5) consiste à calculer le rendement du film $D_{f1}$ correspondant à l'épaisseur $E_1$ en utilisant la fonction définie par l'équation (8), ce qui permet de tenir compte, notamment, de l'influence de l'écran du récepteur. Cette opération a été décrite succinctement ci-dessus.

L'opération (e6) consiste à estimer la lumination $L_f$ reçue par le film depuis le début de la pose en appliquant l'équation suivante :

$$L_f = L_{am} + D_{f1} \times \delta mA.s \quad (9)$$

équation dans laquelle $L_{am}$ est la lumination reçue par le film avant l'opération (e3) et $\delta mA.s$ est le nombre de mA.s débités dans le tube pendant le temps $t'$ et est défini par le produit du courant I de tube par le temps d'intégration S.

L'opération (e7) consiste à calculer la lumination restant à acquérir $L_{ra}$ pour obtenir le noircissement choisi; elle est déterminée par l'équation :

$$L_{ra} = L_{ref} - L_f \quad (10)$$

L'opération (e8) consiste à calculer les mA.s restant à débiter pour obtenir le noircissement choisi et qui est donné par l'équation :

$$mAs_r = L_{ra}/D_{f1} \quad (11)$$

Il est alors possible de déduire le nombre de mA.s débités pendant les calculs, noté $mAs_c$. Alors, les mA.s restant réellement à acquérir, notés $mAs_{ra}$, sont définis par :

$$mAs_{ra} = mAs_r - mAs_c \quad (12)$$



$$mAs_c = I \times t_c \quad (13)$$

avec $t_c$ le temps des calculs.

L'opération (e10) consiste à effectuer un choix : soit arrêter la pose, soit la continuer selon la valeur des mAs restant à débiter ou encore du temps de pose restant à courir, soit recalculer l'estimation de la valeur prévisionnelle du temps de fin de pose.

Le critère de fin de pose pourra être le suivant :

Si la valeur

$$\text{Dif (mA.s)} = \text{mAs}_{ra} - \text{mAs}_{mes} \quad (15)$$

est nulle ou inférieure à une valeur $\text{Val}_0$ fixée, le microprocesseur 19 arrête le rayonnement X par action sur le dispositif d'alimentation 15. Dans le cas contraire, on revient à l'opération (e3).

Il est possible d'envisager un test supplémentaire sur la valeur du temps de pose restant à courir $t_{rc}$ définie par la relation :

$$t_{rc} = \frac{\text{mAs}_{ra}}{I} \quad (14)$$

Ce test supplémentaire consiste à ne pas modifier la valeur de l'estimation $\text{mAs}_{ra}$ dans le cas où $t_{rc}$ est inférieur à une valeur $t_0$. Alors la fin de la pose se termine en boucle ouverte en ne poursuivant que les opérations de fin de pose, c'est-à-dire la décrémentation du nombre de mA.s débités et arrêt de la pose lorsque ce nombre devient inférieur ou égal à zéro. Une valeur possible de $t_0$ est une valeur sensiblement égale à l'intervalle de temps séparant deux mesures correspondant de l'opération (e3). Ainsi, dans ce cas, l'opération (e10) comporte deux tests :

- un premier test sur $\text{mAs}_{ra}$ décidant si on arrête ou non la pose,
- puis un test sur $t_{rc}$ pour décider si on entreprend une nouvelle estimation des mAs restant à débiter ou si la valeur $\text{mAs}_{ra}$ reste figée jusqu'à la fin de la pose. Dans ce dernier cas, le test de fin de pose se fera périodiquement avec la valeur $\text{mAs}_{ra}$.

Par ailleurs, les opérations d'estimation du temps restant à courir et celle de coupure de la pose peuvent être découplées afin d'affiner encore la précision de l'exposeur. Ainsi, le procédé se décompose de la manière suivante : une tâche T.E. destinée à estimer les mA.s restant à débiter avant la fin de la pose et une tâche T.C. de coupure de la pose. Ce sont deux tâches indépendantes qui se déroulent en parallèle.

La tâche d'estimation T.E. des mA.s restant à débiter est constituée des opérations (e3) à (e8) auxquelles s'ajoute une opération (e'9) de conversion des mA.s en un signal dans les unités de la cellule 12 tel que :

$$\text{CE}_{cible} = \text{mAs}_{ra} \times D_c \quad (16)$$

Cette tâche d'estimation T.E. est renouvelée périodiquement pendant la pose, par exemple aux instants $t_1, t_2, \ldots t_n$ qui sont des instants de mesure séparés par une durée qui est au moins égale au temps de calcul $t_c$. A la fin de la tâche d'estimation T.E., la valeur cible $\text{CE}_{cible}$ de la tâche de coupure T.C. est actualisée. Cette actualisation doit tenir compte du signal reçu par la cellule détectrice 12 entre l'instant de mesure au début de l'opération (e3) et l'instant de l'actualisation de la valeur $\text{CE}_{cible}$ à la fin de l'opération T.C.

La tâche de coupure de la pose T.C. est une tâche consistant à décrémenter une valeur donnée ou cible en fonction du signal réellement reçu par cette cellule. Cette tâche coupe la pose dès que la valeur $\text{CE}_{cible}$ devient inférieure ou égale à $\text{Val}_0$, égale à zéro par exemple.

Ainsi, le fonctionnement de la tâche T.C. se résume aux opérations suivantes :

(f1) mesure du signal intégré $M_m$ par la cellule 12 après un certain temps $t_{TC}$;

(f2) décrémentation de cette valeur à la valeur cible :

$$(\text{CE}_{cible} - M_m)$$

(f3) arrêt de la pose lorsque $(\text{CE}_{cible} - M_m)$ est inférieure à $\text{Val}_0$ sinon retour en (f1).

Le procédé qui vient d'être décrit fonctionne correctement dans la mesure où il n'y a pas d'écart à la loi de réciprocité pour le récepteur 17 et la cellule de détection 12. S'il n'en est pas ainsi, il faut compléter les opérations (e6) et (e8) pour en tenir compte et déterminer un coefficient de correction par des mesures et calculs particuliers. Ce coefficient de correction est introduit dans les équations (9) et (11) où interviennent la lumination et le rendement du film.

C'est ainsi que les formules (9) et (11) deviennent :

$$L_f = L_{am} + D_{f1} \times \delta \text{mA.s/CNRD (débit - film)} \quad (9')$$

$$\text{mAs}_{ra} = \frac{L_{ra}}{D_{f1}} \text{CNRD (débit - film)} \quad (11')$$

avec

$$\text{débit - film} = D_{f1} \times I \quad (17)$$

CNRD est la fonction représentant l'effet de non réciprocité exprimé en fonction du débit photonique sur le film.

La fonction CNRD est obtenue par le procédé qui fait l'objet de la présente demande de brevet. Ce procédé consiste d'abord à déterminer les coefficients de non réciprocité du film en fonction de la durée de pose $t_i$ et notés CNRT $(t_i)$. Cette fonction CNRT est déterminée expérimentalement et peut être représentée par une fonction analytique.

De manière plus précise, le procédé consiste à déterminer pour diverses valeurs $\text{IR}_i$ de l'intensité du rayonnement, la valeur $t_i$ du temps d'exposition nécessaire pour obtenir une densité optique $\text{DO}_{refo}$ du film fixée, par exemple $\text{DO}_{refo} = 1$, et à relever les valeurs fournies par le circuit intégrateur 16 pour les différents temps de

pose $t_i$, valeurs que l'on appellera M $(t_i)$.

Ces valeurs sont comparées à une valeur de référence M $(t_{ref})$, qui est par exemple celle correspondant à un temps de pose d'une seconde, en calculant le rapport

$$\frac{M\ (t_i)}{M\ (t_{ref})} \quad (29)$$

C'est ce rapport qui détermine le coefficient de non réciprocité en temps CNRT $(t_i)$ pour le temps de pose $t_i$. Une autre manière pour déterminer ces coefficients CNRT $(t_i)$ sera décrite ultérieurement.

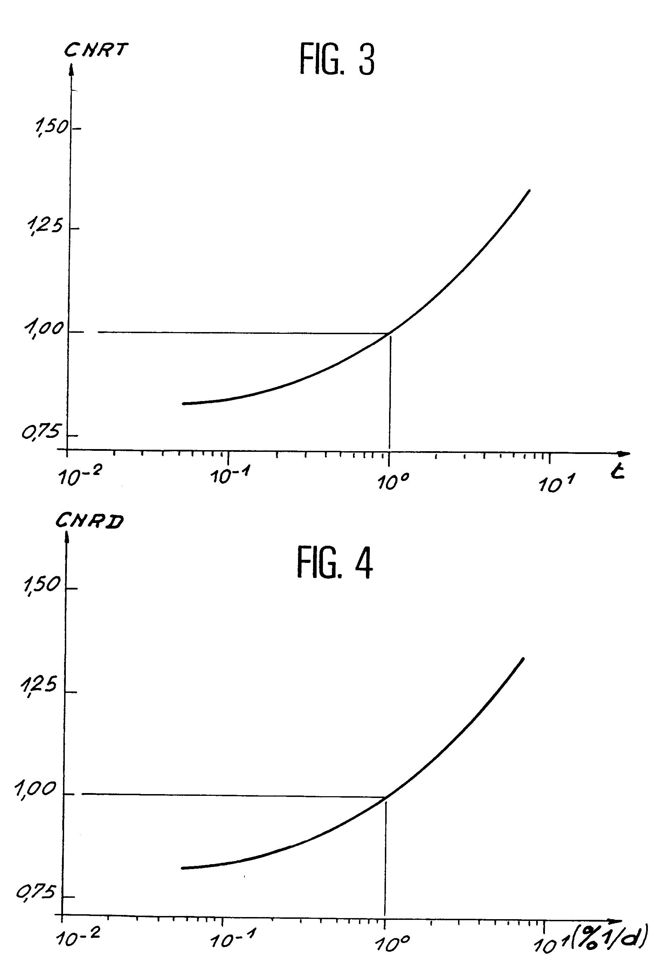

Ces coefficients CNRT $(t_i)$ sont reliés entre eux en fonction du temps de pose par la courbe de la figure 3 dans le cas, par exemple, d'une densité optique $DO_{refo} = 1$ et un temps de pose de référence $t_{ref} = 1$ seconde. Cette courbe montre que la lumination nécessaire pour atteindre la densité optique désirée croit avec le temps de pose. C'est ainsi que, dans cet exemple, le rapport entre les énergies pour les deux temps de pose de 50 ms et 6,5 s est de l'ordre de 1,6.

La courbe de la figure 3 peut être modélisée à l'aide d'une fonction de la forme :

$$CNRT\ (t_i)\ =\ A_o\ +\ A_1 \log t\ +\ A_2\ [\log t]^2 \quad (18)$$

dont les paramètres $A_o$, $A_1$ et $A_2$ sont estimés à partir des points de mesure par une procédure d'estimation aux moindres carrés.

Dans le principe, l'effet Schwarzschild que l'on prend en compte dans les équations (9') et (11') pourrait être modélisé par la fonction CNRT. L'intérêt d'utiliser la fonction CNRD indexée en débit est que l'on peut prendre en compte des variations du courant anodique. Donc, un exposeur automatique qui utilise la fonction CNRD suivant les équations (9') et (11') a, par exemple, pour avantage que le tube peut fonctionner en charge décroissante.

Pour passer des coefficients CNRT (t) indexés en temps aux coefficients CNRD (d) indexés en débit, il faut tenir compte du fait que les coefficients CNRT (t) ont été déterminés par des mesures à temps de pose variable sans connaître nécessairement les valeurs du débit photonique sur le film. Si l'on mesure pour chaque temps de pose $t_i$ le débit-film $d_i$, la valeur du coefficient CNRD $(d_i)$ pour $d_i$ sera égale à celle du coefficient CNRT $(t_i)$ pour le temps de pose $t_i$ correspondant selon la relation :

$$CNRD\ (d_i)\ =\ CNRT\ (t_i) \quad (19)$$

Ces différentes valeurs de CNRD $(d_i)$ sont reliées entre elles par une courbe (figure 4) en fonction de l'inverse 1/d du débit. Cette courbe peut être modélisée à l'aide d'une fonction de la forme :

$$CNRD\ (d)\ =\ A'_o\ +\ A'_1 \log 1/d\ +\ A'_2\ [\log 1/d]^2 \quad (20)$$

Les valeurs $d_i$ peuvent ne pas être données par l'étalonnage, surtout parce qu'elles sont exprimées dans l'unité de mesure de la cellule 12 qui n'est pas nécessairement celle utilisée dans l'étalonnage. Ainsi, en général, il faut relier les valeurs $d_i$ aux valeurs connues $t_i$ par la relation :

$$L_{ref} \times CNRT\ (t_i)\ =\ d_i \times t_i \quad (21)$$

ou encore :

$$d_i\ =\ \frac{L_{ref} \times CNRT\ (t_i)}{t_i} \quad (22)$$

On rappelle ici que $L_{ref}$ est la lumination que reçoit le film dans des conditions radiologiques fixées et connues lorsque le film atteint un noircissement donné et que l'effet de non réciprocité est corrigé.

Pour finaliser la définition de la fonction CNRD, il reste à exposer la méthode d'évaluation de la lumination de référence.

Cette méthode est décrite dans la demande de brevet précitée (EP-A-0465362) et intitulée : PROCEDE D'ESTIMATION ET D'ETALONNAGE DE LA LUMINATION RECUE PAR UN FILM RADIOGRAPHIQUE.

La lumination de référence dépend de la densité optique que l'on souhaite obtenir sur le film. Pour déterminer cette lumination, la première étape est de réaliser un sensitogramme du type de film utilisé, ensuite il faut réaliser un cliché dans des conditions radiologiques déterminées avec un étalon d'épaisseur connue.

Ces conditions radiologiques déterminées sont, par exemple,

- une densité optique de référence $DO_{refo}$ choisie en fonction des habitudes du praticien, par exemple $DO_{refo} = 1$
- un étalon d'épaisseur $E_o$,
- une tension d'alimentation $V_o$,
- une valeur du temps de pose $t_o$,
- une valeur du produit $I_o \times t_o$,

Pour ce cliché, on mesure la densité optique $DO_m$ ainsi que les valeurs $M_o$, $I_o$, $t_o$, ce qui permet de calculer l'épaisseur équivalente $E_p$ à l'aide de la formule (7). On calcule ensuite le rendement $D_{fo}$ sur le film à l'aide de la formule (6), ce qui permet de calculer la lumination reçue par le film $L_{film}$ par la formule :

$$L_{film}\ =\ D_{fo} \times I_o \times t_o \quad (23)$$

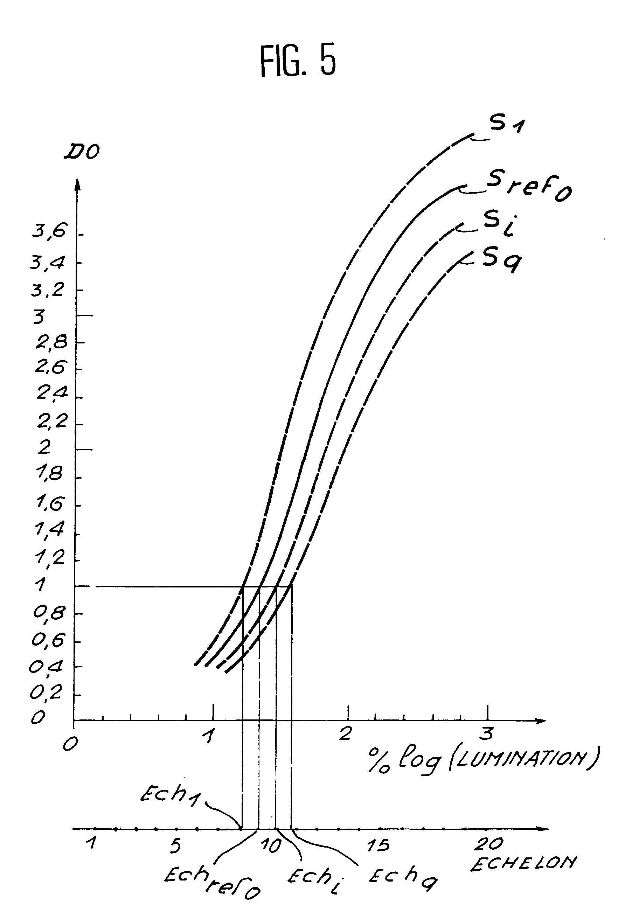

La densité optique de référence $DO_{refo}$ permet de calculer l'échelon d'éclairement $Ech_{ref}$ correspondant à $DO_{refo}$ sur la courbe sensitométrique du film utilisé (figure 5), cette courbe ayant été tracée à l'aide d'un sensitographe et d'un densitomètre. Ceci permet de tenir compte des caractéristiques de la machine à développer qui est utilisée. La courbe est enregistrée, par exemple, sous la forme d'une fonction dans le microprocesseur 19 (fig. 1).

La densité optique mesurée $DO_m$ permet de calculer l'échelon de mesure $Ech_m$ qui est la valeur de l'échelon d'éclairement correspondant à $DO_m$ sur la courbe sensitométrique (fig. 5).

Avec les valeurs $L_{film}$ de la lumination sur le film, de l'échelon de référence $Ech_{ref}$ et de l'échelon de mesure $Ech_m$, il est possible de calculer la lumination de référence $L_{ref}$ pour obtenir la densité optique $DO_{refo}$ en utilisant l'équation qui définit le changement d'échelle entre la lumination et l'échelon d'éclairement de l'axe des abscisses de la courbe sensitométrique (figure 5), soit :

$$Ech_m = Ech_{ref} + K \cdot \log_{10} \left[ \frac{L_{film}}{L_{ref}} \right] \qquad (24)$$

De cette équation (24), on tire :

$$L_{ref} = L_{film} \times exp \left[ \log_{10} \left[ \frac{Ech_{ref} - Ech_m}{K} \right] \right] \qquad (25)$$

avec
$$k = 2/\log_{10}(2) \qquad (26)$$

La constante sensitométrique K correspond à l'échelle retenue pour les échelons d'éclairement.

La valeur $L_{ref}$ dépend de $t_o$ à travers Lfilm par les équations (23) et (25). Ainsi, la valeur $L_{ref}$ est sensible aux effets de non réciprocité du film. Pour corriger l'influence de la non réciprocité sur la valeur de $L_{ref}$, il suffit d'utiliser dans l'équation (23), la valeur $L_{film}$ définie par :

$$L_{film} = \frac{D_f \times I_o \times t_o}{CNRT(t_o)} \qquad (23')$$

Cette lumination de référence $L_{ref}$ est celle qui doit être utilisée dans l'équation (10) pour obtenir la densité optique de référence $DO_{refo}$, par exemple $DO_{refo} = 1$, et la formule (25) montre qu'elle dépend, notamment, de la différence entre l'échelon de référence et l'échelon de mesure.

La connaissance de la lumination reçue par le film permet de connaître $d_i$ par l'application de la formule (22) et à en déduire CNRD $(d_i)$ par la formule (20).

Pour une densité optique du film radiographique autre que $DO_{refo} = 1$, il est nécessaire d'effectuer de nouveau les opérations décrites ci-dessus de manière à déterminer les nouvelles valeurs de CNRT $(t_i)$ et de $L_{ref}$.

Afin de simplifier ces opérations, les coefficients CNRT $(t_i)$ peuvent être obtenus en effectuant les opérations suivantes :

(g1) réalisation à l'aide d'un sensitographe à temps variable, d'un premier sensitogramme $S_{refo}$ (figure 5) lorsque le temps de pose est réglé pour un temps de référence $t_{ref}$;

(g2) réalisation à l'aide du même sensitographe à temps variable, de q sensitogrammes $S_1$ à $S_q$ (figure 5) pour q temps de pose différents $t_i$;

(g3) Choix d'une densité optique de référence $DO_{refo}$, par exemple $DO_{refo} = 1$

(g4) Mesure sur chaque sensitogramme, l'échelon d'éclairement $Ech_{refo}$, $Ech_1...Ech_i...Ech_q$ correspondant à la densité optique $DO_{refo}$ ($DO_{refo} = 1$);

(g5) Calcul du coefficient CNRT $(t_i)$ par l'équation :

$$CNRT(t_i) = exp \left[ \log_{10} \left[ \frac{Ech_{refo} - Ech_i}{K} \right] \right] \qquad (28)$$

Si le praticien décide de travailler à une densité optique différente, il est proposé, afin d'éviter l'étalonnage décrit ci-dessus, d'utiliser la densité optique corrigée volontairement pour le noircissement $DO_{cvn}$. Alors la lumination de référence $L_{ref}$, utilisée dans l'équation (10) doit être remplacée par la lumination corrigée $L_{cvn}$ qui s'exprime par :

$$L_{cvn} = L_{ref} \times exp[CVN/\Gamma \times P \times Log(10)] \quad (27)$$

où

- CVN est la correction volontaire de noircissement exprimée par un nombre entier de -10 à + 10 par exemple,
- P est le pas en densité optique, par exemple 0,1,
- $\Gamma$ est la pente de la partie linéaire de la courbe sensitométrique (figure 5).

Le procédé qui vient d'être décrit montre que sa mise en oeuvre nécessite un certain nombre d'étalonnages qui sont, en résumé, les suivants :

(a) l'étalonnage du système radiologique de manière à déterminer les modèles analytiques

$$D_{se} = f'(V_m, E_p) \quad (4)$$

avec cassette sans écran et

$$D_c = f''(V_m, E_p) \quad (6)$$
$$E_p = g''(V_m, D_c) \quad (7)$$

avec cassette et écran;

La différence $D_f = (D_{se} - D_c)$ (équation (8)) permettra de déduire le rendement absorbé par l'écran;

(b) l'étalonnage du film de manière à déterminer la loi de non réciprocité CNRT (t) exprimée en fonction du temps ; cette loi sera utilisée pour déterminer la loi de non réciprocité CNRD (d) exprimée en fonction du débit;

(c) l'étalonnage de la lumination de référence $L_{ref}$. Ces différents étalonnages ayant été effectués, les différentes opérations proprement dites du procédé sont les suivantes :

(d) choix, par le praticien, de la valeur de noircissement ou de la valeur de la correction volontaire de noircissement de manière à déterminer la lumination cible $L_{cvn}$ que doit recevoir le film dans des conditions de référence fixées pour arriver au noircissement (ou densité optique) choisi. La lumination $L_{cvn}$ est calculée à partir de l'équation (27) où la lumination $L_{ref}$ est déterminée par l'étalonnage (c) et les équations (25) et (26).

(e1) mise en place de l'objet à radiographier;

(e2) déclenchement du début de la pose par le praticien;

(e3) mesure après un temps t' du rendement $D_{c1}$ au niveau de la cellule (12);

(e4) mesure de l'épaisseur équivalente $E_1$ par l'équation (7);

(e5) calcul du rendement $D_{f1}$ au niveau du film pour l'épaisseur $E_1$ par l'équation (8);

(e6) calcul de la lumination $L_f$ reçue par le film par l'équation :

$$L_f = L_{am} + D_{f1} \times \delta mA.s/CNRD \text{ (débit - film)} \quad (9')$$

(e7) calcul de la lumination $L_{ra}$ restant à acquérir pour obtenir le noircissement (ou densité optique) choisi par l'équation

$$L_{ra} = L_{cvn} - L_f \quad (10')$$

(e8) calcul prévisionnel des mA.s restant à débiter $mAs_{ra}$ pour obtenir le noircissement (ou densité optique) par l'équation :

$$mAs_{ra} = L_{ra}/D_{f1} \times CNRD \text{ (débit - film)} \quad (11')$$

(e9) mesure des mA.s débités depuis le début de l'opération (e3).

(e10)

- arrêt de la pose lorsque les mA.s mesurés en (e9) sont égaux ou supérieurs à $mAs_{ra}$.
- ou retour à l'opération (e3) lorsque les mA.s mesurés en (e9) sont inférieures à $mAs_{ra}$.

La description du procédé qui vient d'être faite correspond à une certaine configuration du système de radiologie. Dans le cas où ce système peut prendre plusieurs configurations tels que, par exemple, le choix

- du matériau d'anode,
- des dimensions du foyer,
- du filtre de modification du spectre,
- de la collimation,
- de la présence ou absence d'une grille anti-diffusante,
- du type de récepteur image,
- du type de cellule de détection,

il est nécessaire d'effectuer pour chacune de ces configurations des étalonnages (a), (b) et (c). Le nombre de ces étalonnages peut être réduit en tenant compte des similitudes de comportement d'une configuration à l'autre comme cela a été décrit pour l'étalonnage (a) dans la demande de brevet n° 89 07686 déposée le 9 juin 1989.

Lors de la mise en oeuvre du procédé par le praticien, ce dernier définit la configuration dont les caractéristiques sont transmises au microprocesseur (19) de manière que ce dernier utilise les modèles correspondants.

Le procédé selon l'invention a été décrit dans son application à un récepteur 17 du type couple film-écran. Il peut aussi être mis en oeuvre dans le cas d'un récepteur 17 ne comportant qu'un film sensible au rayonnement X. Avec un tel film,

Les étalonnages des opérations (a) et (b) deviennent :

(a') étalonnage du système radiologique de manière à déterminer le modèle analytique

$$D'f = f'''(V_m, E_p) \quad (30)$$

avec le film comme récepteur image.

Les modifications sont les suivantes :

Dans le déroulement du procédé

- (e3) devient (e'3) : mesure après un temps t' du rendement $D'f_1$ au niveau de la cellule 12;
- (e4) et (e5) sont supprimées et
- les opérations (e6) et (e8) sont modifiées de la manière suivante :

(e'6) calcul de la lumination $L'_f$ reçue par le film par l'équation :

$$L'_f = L_{am} + D'_{f1} \times \delta mA.s/CNRD \text{ (débit - film)} \quad (9'')$$

(e7) calcul de la lumination $L'_{ra}$ restant à acquérir pour obtenir le noircissement (ou densité optique) choisi par l'équation

$$L'_{ra} = L_{cvn} - L'_f \quad (10'')$$

(e'8) calcul prévisionnel des mA.s restant à débiter $mAs'_{ra}$ pour obtenir le noircissement (ou densité optique) par l'équation :

$$mAs'_{ra} = L_{ra}/D'_{f1} \times CNRD \text{ (débit - film)} \quad (11'')$$

Les opérations (e9) et suivantes restant sans changement.

Par ailleurs, il faut noter que le sensitographe peut, dans ce cas, être du type à émission X.

En outre, avec un tel récepteur ne comportant pas d'écran renforçateur, la cellule de détection 12 peut être placée, soit derrière le récepteur 17 comme dans le cas du récepteur du type film-écran, soit devant le récepteur 17 si l'énergie du rayonnement le permet.

## Revendications

1. Procédé de détermination de la fonction représentant l'effet de non réciprocité d'un film radiographique dans un système de radiologie prévu pour examiner un objet (13) qui comprend un tube (11) à rayons X dont la tension d'alimentation peut prendre diverses valeurs $V_m$, à variation continue ou discrète, et qui émet un faisceau (14) de rayons X sous forme d'impulsions de durée variable $t_i$ en direction de l'objet à examiner, un récepteur image (17) du rayonnement X ayant traversé l'objet (13) pour réaliser une image dudit objet, une cellule de détection (12) des rayons X ayant traversé l'objet (13) à examiner qui permet de convertir une grandeur physique caractérisant le faisceau de rayons X en un signal de mesure L, un circuit intégrateur (16) qui intègre le signal de mesure L pendant la durée $t_i$ et fournit un signal M et un dispositif de calcul (18) du rendement D donné par le rapport de M par le produit I x $t_i$ (ou mA.s) du courant anodique I du tube par la durée $t_i$ de la pose, le procédé comprenant les opérations suivantes :

    (a) la détermination des coefficients de non réciprocité CNRT ($t_i$) du film exprimés en temps de pose $t_i$ pour une densité optique $D0_{refo}$ dudit film pour diverses valeurs ($IR_i$) de l'intensité du rayonnement;

    (b) la détermination de la lumination de référence $L_{ref}$ reçue par ledit film dans des conditions radiologiques fixées et connues lorsque le film atteint un noircissement donné et que l'effet de non réciprocité est corrigé;

    (c) le calcul des débits photoniques $d_i$ sur ledit film par la formule :

    $$d_i = \frac{L_{ref} \times CNRT (t_i)}{t_i} \quad (22)$$

    (d) la détermination des coefficients de non réciprocité CNRD ($d_i$) exprimés en débit photonique $d_i$ par l'application de la formule :

    $$CNRD (d_i) = CNRT (t_i) \quad (19)$$

2. Procédé selon la revendication 1, caractérisé en ce qu'il comprend, en outre, l'opération suivante :

    (e) la détermination de la fonction de modélisation des coefficients CNRD (d) telle que

    $$CNRD (d) = A'_0 + A'_1 \log 1/d + A'_2 [\log 1/d]^2 \quad (20)$$

    dont les paramètres $A'_0$, $A'_1$ et $A'_2$ sont estimés à partis des valeurs $d_i$ et CNRD($d_i$).

11

**3.** Procédé selon la revendication 1 ou 2, caractérisé en ce que l'opération (a) comprend les opérations suivantes :

($a_1$) la modification du courant de chauffage du tube (11) de manière à obtenir différentes valeurs dudit courant;

($a_2$) le relevé des valeurs $M(t_i)$ fournies par le circuit intégrateur (16) pour différents temps de pose ($t_i$) de manière à obtenir une densité optique $DO_{refo}$ du film fixée;

($a_3$) le calcul du rapport

$$\frac{M\ (t_i)}{M\ (t_{ref})} \quad (29)$$

qui donne le coefficient CNRT ($t_i$) avec M ($t_{ref}$) la valeur M ($t_i$) pour $t_i = t_{ref}$.

**4.** Procédé selon la revendication 1 ou 2, caractérisé en ce que l'opération (a) comprend les opérations suivantes :

(g1) réalisation à l'aide d'un sensitographe à temps variable, d'un premier sensitogramme $S_{refo}$ lorsque le temps de pose est réglé pour un temps de référence $t_{refo}$;

(g2) réalisation à l'aide du même sensitographe à temps variable, de q sensitogrammes $S_1$ à $S_q$ pour q temps de pose différents $t_i$;

(g3) choix d'une densité optique de référence $DO_{refo}$,

(g4) mesure sur chaque sensitogramme, de l'échelon d'éclairement $Ech_{refo}$, $Ech_1...Ech_i...Ech_q$ correspondant à la densité optique $DO_{refo}$

(g5) calcul des coefficients CNRT ($t_i$) par l'équation :

$$CNRT\ (t_i)\ =\ exp\ \left[log_{10}\left[\frac{Ech_{refo}\ -\ Ech_i}{K}\right]\right] \quad (28)$$

avec $K = 2/log_{10}(2)$.

**5.** Procédé selon la revendication 3 ou 4, caractérisé en ce que l'opération (a) comprend en outre l'opération suivante :

- la détermination de la fonction de modélisation des coefficients CNRT (t) telle que :

$$CNRT\ (t)\ =\ A_0\ +\ A_1\ log\ t\ +\ A_2\ [\ log\ t\ ]^2 \quad (18)$$

dont les paramètres $A_0$, $A_1$ et $A_2$ sont estimés à partir des valeurs $t_i$ et $CNRT(t_i)$.

**6.** Procédé selon l'une quelconque des revendications 1 à 5, caractérisé en ce que l'opération (b) consiste en une mesure de ladite lumination $L_{ref}$ par une cellule détectrice.

**7.** Procédé selon l'une quelconque des revendications 1 à 5, caractérisé en ce que la lumination de référence $L_{ref}$ est déterminée par un procédé d'étalonnage qui comprend les opérations suivantes dans le cas où le récepteur image (17) est composé d'au moins un écran renforçateur et d'un film sensible à la lumière de cet écran:

- Réalisation d'un cliché dans des conditions radiologiques déterminées pour une densité optique de référence $DO_{refo}$, un étalon d'épaisseur $E_o$, une tension d'alimentation $V_o$ un temps de pose $t_o$ et une valeur du produit $I_o \times t_o$, $I_o$ étant le courant de tube;

- Mesure du rendement $D_o$;

- Calcul de l'épaisseur équivalente $E_{po}$ par la formule :

$$E_{po}\ =\ g''\ (V_o,\ D_o) \quad (7)$$

- Calcul du rendement $D_{fo}$ sur le film par la formule :

$$D_{fo}\ =\ f'\ (V_o,\ E_{po})\ -\ f''\ (V_o,\ E_{po}) \quad (8)$$

dans laquelle f' et f'' sont des fonctions qui décrivent respectivement le comportement du rendement D en fonction des paramètres tension d'alimentation et epaisseur de l'étalon pour la configuration donnée du système avec le récepteur (17) comprenant l'écran renforçateur et une configuration du système avec le récepteur sans le ou les écrans renforçateurs, g'' etant la fonction inverse de f'',

- Calcul de la luminance $L_{film}$ sur le film par la formule :

$$L_{film}\ =\ D_{fo}\ \times\ I_o\ \times\ t_o \quad (23)$$

- Calcul de l'échelon d'éclairement $Ech_{ref}$ correspondant à la densité optique de référence $DO_{refo}$ à l'aide de la courbe sensitométrique $S_{refo}$;

- Mesure de la densité optique $DO_m$ du cliché obtenu et calcul de l'échelon d'éclairement $Ech_m$ correspondant à $DO_m$ à l'aide de la courbe sensitométrique $S_{refo}$;
- Calcul de la lumination de référence $L_{ref}$ par la formule :

$$L_{ref} = L_{film} \times exp \left[ log_{10} \left[ \frac{Ech_{ref} - Ech_m}{K} \right] \right] \quad (25)$$

avec

$$K = 2/log_{10}(2) \quad (26)$$

8. Procédé selon la revendication 7, caractérisé en ce que pour le calcul de rendement $D_{fo}$ sur le film par l'équation (8), l'épaisseur équivalente $E_{po}$ est remplacée par ($E_{po}$ - (sup.filtre)), (sup.filtre) étant l'épaisseur équivalente due à la filtration additionnelle résultant de l'atténuation entre l'écran renforçateur et la cellule de détection (12).

9. Procédé selon la revendication 7 dans le cas où le récepteur image (17) est composé d'un film sensible au rayonnement X et où la cellule de détection (12) est disposée avant ou après ledit film, caractérisé en ce que le rendement $D_{fo}$ sur le film est calculée par la formule :

$$D'_{fo} = f'''(V_o, E_p) \quad (30)$$

dans laquelle f''' est une fonction qui décrit le comportement du rendement D en fonction des paramètres tension d'alimentation et epaisseur de l'étalon pour la configuration donnée du système.

10. Procédé selon la revendication 9, caractérisé en ce que dans l'équation (30), l'épaisseur $E_p$ est remplacée par ($E_p$ - sup.filtre), (sup.filtre) étant l'épaisseur équivalente due à la filtration additionnelle résultant de l'atténuation entre le film du récepteur (17) et la cellule détectrice (12).

11. Procédé selon l'une quelconque des revendications 7 à 10, caractérisé en ce que la luminance sur le film est calculée par la formule :

$$L'_{film} = \frac{D_{fo} \times I_o \times t_o}{CNRT(t_o)} \quad (23')$$

## Patentansprüche

1. Verfahren zur Bestimmung der die Wirkung der Nichtreziprozität eines Röntgenfilms repräsentierenden Funktion in einem für die Untersuchung eines Gegenstandes (13) vorgesehenen radiologischen System, das versehen ist mit einer Röntgenröhre (11), deren Versorgungsspannung verschiedene kontinuierlich oder diskret sich verändernde Werte $V_m$ annehmen kann und die ein Bündel (14) von Röntgenstrahlen in Form von Impulsen mit veränderlicher Dauer $t_i$ in Richtung des zu untersuchenden Gegenstandes aussendet, einem Bildempfänger (17) für die Röntgenstrahlung, die den Gegenstand (13) durchquert hat, um ein Bild des Gegenstandes zu verwirklichen, einer Detektorzelle (12) für die Röntgenstrahlen, die den zu untersuchenden Gegenstand (13) durchquert haben, mit der eine das Bündel von Röntgenstrahlen kennzeichnende physikalische Größe in ein Meßsignal L umgewandelt werden kann, einer Integratorschaltung (16), die das Meßsignal L während der Dauer $t_i$ integriert und ein Signal M ausgibt, und einer Einrichtung (18) zur Berechnung des Wirkungsgrades D, der durch das Verhältnis von M zum Produkt $I \times t_i$ (oder mA · s) des Anodenstroms I der Röhre mit der Dauer $t_i$ der Aufnahme gegeben ist, wobei das Verfahren die folgenden Operationen enthält:

(a) Bestimmen der Nichtreziprozitäts-Koeffizienten $CNRT(t_i)$ des Films, die für eine optische Dichte $DO_{refo}$ des Films für verchiedene Werte $(IR_i)$ der Strahlungsintensität durch die Aufnahmezeit $t_i$ ausgedrückt werden;

(b) Bestimmen der Referenzbelichtung $L_{ref}$, die von dem Film unter festen und bekannten radiologischen Bedingungen empfangen wird, wenn der Film eine gegebene Schwärzung erreicht und die Wirkung der Nichtrepozität korrigiert ist;

(c) Berechnen der Photonenraten $d_i$ für den Film durch die Formel:

$$d_i = \frac{L_{ref} \times CNRT(t_i)}{t_i} \quad (22)$$

(d) Bestimmen der Nichtreziprozitäts-Koeffizienten CNRD($d_i$), die durch die Photonenrate $d_i$ ausgedrückt werden, durch Anwenden der Formel:

$$CNRD(d_i) = CNRT(t_i). \quad (19)$$

2. Verfahren gemäß Anspruch 1, dadurch gekennzeichnet, daß es außerdem die folgende Operation enthält:
   (e) Bestimmen der Modellfunktion der Koeffizienten CNRD(d), derart, daß

$$CNRD(d) = A'_0 + A'_1 \log\frac{1}{d} + A'_2 \left[\log\frac{1}{d}\right]^2 \qquad (20)$$

wobei die Parameter $A'_0$, $A'_1$ und $A'_2$ anhand der Werte $d_i$ und CNRD($d_i$) geschätzt werden.

3. Verfahren gemäß Anspruch 1 oder 2, dadurch gekennzeichnet, daß die Operation (a) die folgenden Operationen enthält:
   ($a_1$) Modifizieren des Heizstroms der Röhre (11) in der Weise, daß verschiedene Werte des Stroms erhalten werden;
   ($a_2$) Ablesen der Werte M($t_i$), die von der Integratorschaltung (16) für verschiedene Aufnahmezeiten ($t_i$) ausgegeben werden, um so eine feste optische Dichte $DO_{refo}$ des Films zu erhalten;
   ($a_3$) Berechnen des Verhältnisses

$$\frac{M(t_i)}{M(t_{ref})}, \quad (29)$$

das den Koeffizienten CNRT($t_i$) angibt, wobei M($t_{ref}$) der Wert M($t_i$) für $t_i = t_{ref}$ ist.

4. Verfahren gemäß Anspruch 1 oder 2, dadurch gekennzeichnet, daß die Operation (a) die folgenden Operationen enthält:
   (g1) Verwirklichen eines ersten Sensitogramms $S_{refo}$ mit Hilfe eines Sensitometers mit veränderlicher Zeit, wenn die Aufnahmezeit für eine Referenzzeit $t_{refo}$ gesteuert wird;
   (g2) Verwirklichen von q Sensitogrammen $S_1$ bis $S_q$ mit Hilfe desselben Sensitometers mit veränderlicher Zeit für q verschiedene Aufnahmezeiten $t_i$;
   (g3) Wählen einer optischen Referenzdichte $DO_{refo}$,
   (g4) Messen der Beleuchtungsstufe $Ech_{refo}$, $Ech_1$,...,$Ech_i$, ..., $Ech_q$, die der optischen Dichte $DO_{refo}$ entsprechen, für jedes Sensitogramm,
   (g5) Berechnen der Koeffizienten CNRT($t_i$) durch die Gleichung:

$$CNRT(t_i) = \exp\left[\log_{10}\left[\frac{Ech_{refo} - Ech_i}{K}\right]\right], \qquad (28)$$

mit $K = 2/\log_{10}(2)$.

5. Verfahren gemäß Anspruch 3 oder 4, dadurch gekennzeichnet, daß die Operation (a) außerdem die folgende Operation enthält:
   - Bestimmen der Modellfunktion der Koeffizienten CNRT (t), derart, daß:
   $$CNRD(t) = A_0 + A_1 \log t + A_2 [\log t]^2 \quad (18)$$
   wobei die Parameter $A_0$, $A_1$ und $A_2$ anhand der Werte $t_i$ und CNRT($t_i$) geschätzt werden.

6. Verfahren gemäß einem der Ansprüche 1 bis 5, dadurch gekennzeichnet, daß die Operation (b) in einer Messung der Belichtung $L_{ref}$ mittels einer Detektorzelle besteht.

7. Verfahren gemäß einem der Ansprüche 1 bis 5, dadurch gekennzeichnet, daß die Referenzbelichtung $L_{ref}$ durch ein Eichverfahren bestimmt wird, das in dem Fall, in dem der Bildempfänger (17) aus wenigstens einem Verstärkerschirm und einem für das Licht dieses Schirms empfindlichen Film aufgebaut ist, die folgenden Operationen enthält:
   - Verwirklichen einer Aufnahme unter bestimmten radiologischen Bedingungen für eine optische Referenzdichte $DO_{refo}$, ein Normal der Dicke $E_o$, eine Versorgungsspannung $V_o$, eine Aufnahmezeit $t_o$ und einen Wert des Produkts $I_o \times t_o$, wobei $I_o$ der Röhrenstrom ist;

- Messen des Wirkungsgrades $D_o$;
- Berechnen der äquivalenten Dicke $E_{po}$ durch die Formel:

$$E_{po} = g'' (V_o, D_o) \quad (7)$$

- Berechnen des Wirkungsgrades $D_{fo}$ für den Film durch die Formel:

$$D_{fo} = f' (V_o, E_{po}) - f'' (V_o, E_{po}) \quad (8)$$

wobei f' bzw. f'' Funktionen sind, die das Verhalten des Wirkungsgrades D in Abhängigkeit von den Parametern der Versorgungsspannung und der Dicke des Normals für die gegebene Konfiguration des Systems, in der der Empfänger (17) den Verstärkerschirm enthält, bzw. für die Konfiguration des Systems, in der der Empfänger keinen oder keine Verstärkerschirme enthält, beschreiben, und wobei g'' die inverse Funktion von f'' ist.

- Berechnen der Belichtung $L_{film}$ für den Film durch die Formel:

$$L_{film} = D_{fo} \times I_o \times t_o \quad (23)$$

- Berechnen der Beleuchtungsstufe $Ech_{ref}$, die der optischen Referenzdichte $DO_{refo}$ entspricht, mit Hilfe der sensitometrischen Kurve $S_{refo}$;
- Messen der optischen Dichte $DO_m$ der erhaltenen Aufnahme und Berechnen der Beleuchtungsstufe $Ech_m$, die $DO_m$ entspricht, mit Hilfe der sensitometrischen Kurve $S_{refo}$;
- Berechnen der Referenzbelichtung $L_{ref}$ durch die Formel:

$$L_{ref} = L_{film} \times \exp\left[ \log_{10}\left[ \frac{Ech_{ref} - Ech_m}{K} \right] \right] \quad (25)$$

mit

$$K = 2/\log_{10}(2) \quad (26)$$

**8.** Verfahren gemäß Anspruch 7, dadurch gekennzeichnet, daß für die Berechnung des Wirkungsgrades $D_{fo}$ für den Film durch die Gleichung (8) die äquivalente Dicke $E_{po}$ durch ($E_{po}$ - (zusätzliches Filter)) ersetzt wird, wobei (zusätzliches Filter) die äquivalente Dicke aufgrund der zusätzlichen Filterung ist, die sich aus der Dämpfung zwischen dem Verstärkerschirm und der Detektorzelle (12) ergibt.

**9.** Verfahren gemäß Anspruch 7 für den Fall, in dem das Empfängerbild (17) aus einem für die Röntgenstrahlung empfindlichen Film gebildet ist und in dem die Detektorzelle (12) vor oder hinter dem Film angeordnet ist, dadurch gekennzeichnet, daß der Wirkungsgrad $D_{fo}$ für den Film durch die folgende Formel berechnet wird:

$$D'_{fo} = f''' (V_o, E_p), \quad (30)$$

in der f''' eine Funktion ist, die das Verhalten des Wirkungsgrades (D) in Abhängigkeit von den Parametern der Versorgungsspannung und der Dicke des Normals für die gegebene Konfiguration des Systems beschreibt.

**10.** Verfahren gemäß Anspruch 9, dadurch gekennzeichnet, daß in der Gleichung (30) die Dicke $E_p$ durch ($E_p$ - (zusätzliches Filter)) ersetzt ist, wobei (zusätzliches Filter) die äquivalente Dicke aufgrund der zusätzlichen Filterung ist, die sich aus der Dämpfung zwischen dem Empfängerfilm (17) und der Detektorzelle (12) ergibt.

**11.** Verfahren gemäß einem der Ansprüche 7 bis 10, dadurch gekennzeichnet, daß die Leuchtdichte für den Film durch die Formel berechnet wird:

$$L'_{film} = \frac{D_{fo} \times I_0 \times t_0}{CNRT(t_0)}. \quad (23')$$

## Claims

**1.** A method for the determination of the function representing the non-reciprocity of a radiographic film in a radiology system provided for examining an object (13) which comprises an x-ray tube (11) whose supply voltage may assume different values $V_m$, with continuous or discrete variation, and which emits a beam (14) of x-rays in the form of pulses with a variable duration $t_i$ toward the object (13) to be examined, an image receiver (17) for x-ray radiation which has passed through the object (13) in order to produce an image of the said object, an detection cell (12) for x-ray radiation which has passed through the object (13) to be examined and which permits the conversion of a physical quantity characteristic of the x-ray beam into a measurement signal L, an integrating circuit (16) which integrates the measurement signal L during the duration $t_i$ and supplies a measurement signal M and a calculation device (18) for the yield D equal to the ratio of M divided by the product $I \times t_i$ (or mA.s) of the anode current I of the tube times the duration $t_i$ of the exposure, such method comprising the following operations:

(a) the determination of the coefficients of non-reciprocity CNRT $(t_i)$ of the film expressed in the exposure time $t_i$ for an optical density $DO_{refo}$ of the said film for various values $(IR_i)$ of the intensity of radiation,

(b) the determination of the reference illumination $L_{ref}$ received by the said film under predetermined radiology conditions and known when the film attains a predetermined blackening and when the effect of non-reciprocity is corrected,

(c) the calculation of the photon rates $d_i$ on the said film by the equation

$$d_i = \frac{L_{ref} \times CNRT(t_i)}{t_i}; \quad (22)$$

(d) the determination of non-reciprocity coefficients of CNRD $(d_i)$ expressed as a photon rate $d_i$ by the application of the equation

$$CNRD (d_i) = CNRT (t_i) \quad (19)$$

2. The method as claimed in claim 1, characterized in that it furthermore comprises the following operation:

(e) the determination of the modeling function of the coefficients CNRD (d) such that

$$CNRD (d) = A'_0 + A'_1 \log 1/d + A'_2 [\log 1/d]^2 \quad (20)$$

whose parameters $A'_0$, $A'_1$ and $A'_2$ are estimated on the basis of the values $d_i$ and CNRD $(d_i)$.

3. The method as claimed in claim 1 or in claim 2, characterized in that the operation (a) comprises the following operations:

($a_1$) the modification of the heating current of the tube (11) in such a manner that different values of the said current are obtained,

($a_2$) taking the values M $(t_i)$ furnished by the integrating circuit (16) for different exposure times $(t_i)$ in such a manner as to obtain a predetermined optical density $DO_{refo}$ of the film,

($a_3$) calculation of the ratio

$$\frac{M(t_i)}{M(t_{ref})}, \quad (29)$$

which gives the coefficient CNRT $(t_i)$ with M $(t_{ref})$ the value M $(t_i)$ being $t_i = t_{ref}$.

4. The method as claimed in claim 1 or claim 2, characterized in that the operation comprises the following operations:

(g1) the production with the aid of a variable time sensitographic instrument of a first sensitogram $s_{refo}$, when the exposure time is regulated for a reference time $t_{refo}$;

(g2) the production with the aid of the same variable time sensitographic instrument of q sensitograms $S_1$ to $S_q$ for q different exposure times $t_i$;

(g3) the selection of a reference optical density $DO_{refo}$,

(g4) the measurement using each sensitogram of the illumination increment $Ech_{refo}$, $Ech_1$,...$Ech_i$...$Ech_q$ corresponding to the optical density $DO_{refo}$,

(g5) the calculation of the coefficient CNRT $(t_i)$ by the equation:

$$CNRT(t_i) = \exp\left[\log_{10}\left[\frac{Ech_{refo} - Ech_i}{K}\right]\right], \quad (28)$$

wherein $K = 2/\log_{10}(2)$.

5. The method as claimed in claim 3 or 4, characterized in that the operation (a) furthermore comprises the following operation:

- the determination of the modeling function of the coefficients CRNT (t) such that:

$$CNRT (t) = A_0 + A_1 \log t + A_2 [\log t]^2 \quad (18)$$

whose parameters $A'_0$, $A'_1$ and $A'_2$ are estimated on the basis of the values $t_i$ and CNRD $(t_i)$.

6. The method as claimed in any one of the claims 1 through 5, characterized in that the operation (b) consists of a measurement of the said lumination $L_{ref}$ by a detector cell.

7. The method as claimed in any one of the claims 1 through 5, characterized in that the reference lumination $L_{ref}$ is determined by a calibration method which comprises the following operations in a case in which the image receiver (17) is made up of at least one intensifier screen and of a film sensitive to the light of such screen:

- the production of an exposure under predetermined radiological conditions for a reference optical density, for example $DO_{refo}$, of a standard of a thickness $E_o$, a supply voltage $V_o$, an exposure time of $t_o$ and a value of the product $I_o \times t_o$, $I_o$ being the tube current;
- measurement of the yield $D_o$;
- calculation of the equivalent thickness $E_{po}$ in accordance with the equation
$$E_{po} = g''(V_o, D_o) \quad (7)$$
- calculation of the yield $D_{fo}$ for the film by the equation:
$$D_{fo} = f'(V_o, E_{po}) - f''(V_o, E_{po}) \quad (8)$$
wherein f' and f'' are functions which respectively describe the variation of the yield D as a function of the parameters supply voltage and thickness of the standard for the predetermined configuration of the system, said receiver (17) comprising the intensifier screen and with a configuration of the system for reception without the intensifier screen or screens, g'' being the inverse function of f''.
- calculation of the luminance $L_{film}$ for the film by the equation
$$L_{film} = D_{fo} \times I_o \times t_o \quad (23)$$
- calculation of the illumination increment $Ech_{ref}$ corresponding to the reference optical density $DO_{refo}$ with the aid of the sensitometric curve $S_{refo}$;
- measurement of the optical density $DO_m$ of the exposure obtained and calculation of the illumination increment $Ech_m$ corresponding to $DO_m$ with the aid of the sensitometric curve $S_{refo}$, and
- calculation of the reference illumination $L_{ref}$ by the equation:

$$L_{ref} = L_{film} \times \exp\left[\log_{10}\left[\frac{Ech_{ref} - Ech_m}{K}\right]\right] \quad (25)$$

wherein $K = 2/\log_{10}(2) \quad (26)$.

8. The method as claimed in claim 7, characterized in that for the calculation of the yield $D_{fo}$ for the film using the equation (8), the equivalent thickness $E_{po}$ is replaced by ($E_{po}$ - (sup.filter)), (sup.filter) being the equivalent thickness due to additional filtration resulting from attenuation between the intensifier screen and the detection cell (12).

9. The method as claimed in claim 7, in a case wherein the image receiver (17) is made up of an x-ray sensitive film and wherein the detection cell (12) is arranged before or behind the film, characterized in that the yield $D_{fo}$ for the film is calculated by the following equation
$$D'_{fo} = f'''(V_o, E_p) \quad (30)$$
wherein f''' is a function which describes the variation of the yield D as a function of the parameters supply voltage and thickness of the standard for the predetermined configuration of the system.

10. The method as claimed in claim 9, characterized in that in equation (30), the thickness $E_p$ is replaced by ($E_p$ - sup.filter), (sup.filter) being the equivalent thickness due to the additional filtration resulting from attenuation between the film of the receiver (17) and the detection cell (12).

11. The method as claimed in any one of the claims 7 through 10, characterized in that the luminance on the film is calculated by the following equation:
$$L'_{film} = \frac{D_{fo} \times I_0 \times t_0}{CNRT(t_0)}. \quad (23')$$

FIG. 1

FIG. 2

FIG. 3

FIG. 4

# FIG. 5